# EUROPEAN PATENT APPLICATION

(11) **EP 3 076 066 A1**
(43) Date of publication of application: **05.10.2016**
(21) Application number: 16161458.1
(22) Date of filing: 21.03.2016
(51) Int. Cl.: F17C 13/02, A61M 16/00

(54) **OXYGEN STORAGE AND SUPPLY UNIT, OXYGEN SUPPLY SYSTEM AND METHOD OF OXYGEN SUPPLY**

(30) Priority: 01.04.2015 GB 201505677
(71) Applicant: Linde Aktiengesellschaft, 80331 München (DE)
(72) Inventor: Schmehl, Wolfgang, 82031 Grünwald (DE); Häussermann, Sabine, 82069 Neuried (DE); Bause, Matthias, 82205 Gilching (DE); Gupta, Raj, Guilford, Yorkshire WF15 7 EW (GB); Jacobsen, Brian, Horsham, Sussex RH12 5YW (GB); Lambert, Piers, Camberley, Surrey GU16 9NW (GB); Perez de Alejo, Rigoberto, Farnham, Surrey GU9 0JE (GB); Thind, Mandip, Southall, UB2 5RS (GB)
(74) Representative: m patent group

(57) **Abstract**

An oxygen storage and supply unit (10) comprising a pressure vessel (11) adapted to store a volume of pressurized oxygen, at least one controllable outlet valve module (13) adapted to adjust an oxygen flow from the pressure vessel (11), a valve control module (14) adapted to regulate the at least one outlet valve module (13) on the basis of a control signal, a human user interface module (19) adapted to indicate data relating to the oxygen storage and supply unit (10), a central control unit (15), a communications module (16), and a housing module (12) at least partially enclosing the at least one controllable outlet valve module (13), the valve control module (14), the central control unit (15), the communications module (16), and the human user interface module (19) is provided. The oxygen storage and supply unit (10) is adapted to provide the control signal to the valve control module (14) on the basis of an oxygen saturation value indicating an oxygen saturation of the blood of a patient (1) which is transmitted to the oxygen storage and supply unit (10) and which is received in the oxygen storage and supply unit (10) via the communications module (16). An oxygen supply system (100) and a method of oxygen supply is also part of the present invention.

## Description

The present invention relates to an oxygen storage and supply unit, to an oxygen supply system, and to a method of oxygen supply according to the pre-characterising clauses of the independent claims.

### Prior art

Oxygen therapy is by far the most frequently administered emergency treatment in hospital and during prehospital care. Oxygen therapy primarily is intended to prevent hypoxia in patients. However, in some respiratory patients, e.g. patients suffering from chronical obstructive pulmonary disease (COPD), also hyperoxia induced by oxygen therapy may pose severe health risks. In such patients, the elimination of carbon dioxide may be compromised, causing hypercapnia. Hypercapnia triggers a higher breathing frequency and therefore an even increased oxygen intake.

According to Aubier et al., Am. Rev. Respir. Dis. 122, 1980, 191-199, about 20% of COPD patients can be diagnosed with respiratory acidosis on arrival in hospitals, respiratory acidosis being correlated with the partial pressure of arterial oxygen and increased the risk of hospital death. Plant et al., Thorax 55, 2000, 550-554, report an increased mortality in COPD patients at oxygen saturation values between 88% and 92%. The mortality rate was significantly reduced in patients with confirmed COPD treated with adjusted oxygen flows. Especially in such patients, oxygen therapy thus should be tailored ("titrated") to the oxygen requirements of the patient to provide an effective therapy and to avoid adverse effects of hypo- and hyperoxia.

The classical method of oxygen titration is manual. A healthcare professional (HCP) periodically reads the oxygen saturation value and manually adjusts the oxygen flow from the oxygen source to the patient via an oxygen flow meter ("rotameter"). The flow meter is typically connected to a wall outlet in a ward or an oxygen cylinder. In manual oxygen titration, frequent or permanent attendance of healthcare professionals is required. As the oxygen saturation value is read only periodically, the oxygen supply can not be adapted in case of short-term variabilities in oxygen saturation. Furthermore, manual methods may suffer from human error.

Also automated oxygen supply devices capable of automatically adjusting oxygen supply to a patient on the basis of a oxygen saturation value provided by an oximeter have been suggested, e.g. in US 3,734,091 A. Such automated systems may, in more sophisticated versions, include a closed-loop control wherein oxygen saturation values are read continuously or at least in a higher frequency than in a manual method, and wherein an oxygen supply is adjusted on the basis of these values. As e.g. reported by Lellouche, Can. Respir. J. 20, 2013, 259-261, however, closed-loop oxygen titration devices are still not in common use in medicine. This may be due to lack of user-friendliness of systems according to the prior art.

The object of the present invention is therefore to improve automated oxygen titration methods and devices, especially in view of ease of use, and consequently provide for a higher degree of acceptance of automated oxygen titration in clinical contexts.

### Disclosure of the invention

According to the present invention, an oxygen storage and supply unit, an oxygen supply system, and a method of medical oxygen supply comprising the features of the independent claims is disclosed. Preferred embodiments are subject of the dependent claims and of the description that follows.

It should be noted that, if the present application uses the term "oxygen," this term also includes oxygen-rich fluids which do not entirely consist of oxygen. The term "oxygen" according to the present application, therefore, also includes fluids which are enriched in oxygen, "enriched" meaning an oxygen content which is above the oxygen content of atmospheric air, i.e. above 25%, 50% and/or 75% by volume.

If, here and in the following, reference is made to units, modules and devices etc. which are "adapted" to perform a certain function, such units, modules and devices comprise means that are specifically designed, shaped, or operable to perform this function. Such means may be implemented as hardware or software means.

If, in the following, reference is made to an oxygen storage and supply unit, the explanations referring to such a unit are obviously equally valid for or an oxygen supply system comprising such an oxygen storage and supply unit and vice versa. Repeated explanations are omitted for conciseness.

### Advantages of the invention

The present invention is based on the finding that an "intelligent" oxygen cylinder may be supplemented with functionality that implements titrated oxygen supply to a patient. An intelligent oxygen cylinder is a pressurized vessel ("cylinder") for oxygen which is equipped with an integrated unit for oxygen flow and/or pressure control, especially embodied as a so-called valve-integrated pressure regulator (VIPR). The pressurized vessel, together with the integrated unit for oxygen flow and/or pressure control, thus forms a unit that both stores and supplies oxygen and is thus referred to as "oxygen storage and supply unit" in the following. As an integrated unit, the elements implementing the above functionality are at least partially enclosed in a common housing. Thus, the oxygen storage and supply unit forms one single device that can easily be moved around and exchanged, e.g. when oxygen is exhausted, without medical staff having to replace several elements of a classical oxygen supply system. In such an integrated unit, faulty operation by a user, e.g. leading to oxygen leaks and consequently fire hazards, may be almost excluded.

Valve integrated pressure regulators are known per se. When using gas cylinders, the pressure of the compressed gas, typically 200 bar, has to be reduced to a pressure suitable for the need of the patient by means of a pressure regulator. Classically, valve arrangements including at least two separate valves were used to that purpose, including an on/off valve directly connected to the gas cylinder and a flow-regulation valve downstream thereof. In such classical valve arrangements, the pressure of the compressed gas in the gas cylinder and the pressure of the oxygen delivery line can be measured via separate manometers and the gas flow can be adapted accordingly.

As such valve arrangements are, however, rather bulky and their operation is often considered laborious or at least unintuitive, the valve-integrated pressure regulators have become increasingly popular during the recent years. A valve-integrated pressure regulator may also comprise the two valves mentioned above, i.e. an on/off valve directly connected to the gas cylinder and a flow regulation valve downstream thereof. However, also valve-integrated pressure regulators including only one valve which has the function of an on/off valve and a flow regulation valve can be used. Such arrangements are enclosed within the scope of the present invention. These valves are, however, typically integrated together with further valves and/or other components into one compact design and may operable via a single device, e.g. a mechanical handle and/or electronic input means.

WO 2012/164240 A2 discloses a valve-integrated pressure regulator comprising a control valve with an aperture for compressed gas and a variable aperture obturator. The aperture obturator is coupled for movement with and by an actuator. To monitor a position of the aperture obturator and, correspondingly, of the opening state of the control valve, a valve position monitor is provided. By monitoring the position of the aperture obturator and of a pressure of the compressed gas in the gas cylinder coupled with the valve integrated pressure regulator, a remaining oxygen supply time can be estimated with high accuracy.

As an "intelligent" oxygen cylinder, the oxygen storage and supply unit which is used according to the present invention includes functionality to provide the user of the oxygen storage and supply unit with information regarding the oxygen storage and supply unit, as indicated below. Especially, the user is provided with information regarding the remaining oxygen supply time. The oxygen storage and supply unit may be integrated into a supply chain of an oxygen supplier, optionally providing for automatic replacement if oxygen is spent or about to be spent.

The oxygen storage and supply unit of the present invention is further provided with functionality that enables for adaptably adjusting an oxygen flow that is provided by the oxygen storage and supply unit to a patient on the basis of an oxygen saturation in the blood of the patient. Consequently, no further devices for that purpose need to be provided, significantly enhancing user-friendliness. Also the functionality that enables for adaptably adjusting the oxygen flow to the patient is preferably formed as an integral part of the oxygen storage and supply unit and thus does not need to be separately exchanged, replaced, etc. by medical staff.

According to the present invention, an oxygen storage and supply is provided. The oxygen storage and supply unit comprises a pressure vessel adapted to store a volume of pressurized oxygen and least one controllable outlet valve module adapted to adjust an oxygen flow from the pressure vessel. As mentioned, the medical oxygen supply unit preferably comprises a valve-integrated pressure regulator. Therefore, besides the at least one controllable outlet valve module further valves may be provided, or the at least one controllable outlet valve module may comprise such further valves.

According to the present invention, the oxygen storage and supply unit further comprises a valve control module adapted to regulate the at least one outlet valve on the basis of a control signal, a human user interface module adapted to indicate data relating to oxygen storage and supply unit, e.g. a remaining time of oxygen supply, a central control unit, a communications module, and a housing module at least partially enclosing the at least one controllable outlet valve module, the valve control module, the central control unit, the communications module, and the human user interface unit. So far, the oxygen storage and supply unit according to the present invention may correspond to the "intelligent cylinders" as known from the prior art mentioned above.

According to the present invention, however, the oxygen storage and supply unit is adapted to provide the control signal to the valve control module on the basis of an oxygen saturation value of a blood of a patient which is provided to the oxygen storage and supply unit. The central control unit of the oxygen storage and supply unit is adapted to provide the control signal to the valve control module on the basis of the oxygen saturation value which is externally transmitted to the oxygen storage and supply unit and which is received in the oxygen storage and supply unit via the communications module. The communications module is thus adapted to receive the oxygen saturation value. Thus, the present invention integrates this functionality into an oxygen storage and supply unit, especially into an "intelligent cylinder."

Oxygen measurement is typically performed non-invasively by pulse oximetry via oximeters. In oximeters for pulse oximetry, typically a sensor is placed on a thin and therefore optically translucent part of the patient's body, usually a fingertip or an earlobe, and light of two wavelengths is passed through this body part to a photodetector. The absorbance of each of the two wavelengths is measured, allowing a determination of the absorbance of the pulsing arterial blood alone without interference of peripheral blood. While pulse oximetry is convenient for the patient, it may suffer from inaccuracies. Therefore, it is, according to the present invention, also possible to use invasive blood gas measurements which are more exact. If possible, also non-invasive blood gas measurements may be performed. Such (invasive or non-invasive) blood gas measurements can also be used to calibrate an oximeter performing pulse oximetry in the present invention. The central control module of the oxygen storage and supply unit of the present invention may be adapted to perform a corresponding calibration and may also be adapted to receive, via the communications module or otherwise, data from blood gas measurements.

In the context of the present invention, the oxygen saturation value is preferably provided by such an oximeter or by an oxygen saturation sensing unit comprising such an oximeter or an oxygen saturation sensing module. The oxygen saturation value may also be provided by an oxygen saturation sensing unit comprising means for invasive or non-invasive blood gas measurement. The oxygen saturation value indicative of the oxygen saturation the blood of the patient may be a raw sensor value which may be communicated to the oxygen storage and supply unit in digital or analogue form or it may be a sensor value processed in the oxygen saturation sensing unit to some extent, e.g. to yield a percent or target value. The oxygen storage and supply unit may be adapted to process oxygen saturation values of different kinds.

According to a particularly preferred embodiment of the present invention, the communications module is adapted for wireless communication according to at least one wireless communication protocol. The wireless communication protocol may be any protocol as known in the prior art, e.g. WIFI (IEEE 802.11a/b/g/n), WPAN (IEEE 802.15.4) or Bluetooth (IEEE 802.15.1). Modifications of such protocols may also be used, e.g. 6LoWPAN or ZigBee. The wireless communication protocol may also be a proprietary protocol specifically adapted for the purposes of the invention. The wireless communication protocol may include encrypted or unencrypted communication.

Advantageously, the oxygen storage and supply unit is further adapted to provide user information via the human user interface module on the basis of oxygen saturation value externally transmitted to the oxygen storage and supply unit, especially based on one or more signals from the central control unit. By using the oxygen storage and supply unit according to the present invention, therefore, additional devices for informing clinical staff or the patient about the oxygen saturation measured may be dispensed of, again contributing to enhanced user-friendliness. As an oximeter or an oxygen saturation sensing unit comprising such an oximeter does not need to be provided with corresponding means to provide user information, it may be constructed in a compact way and causes minimum annoyance to the patient and to clinical staff. Dispensing of an additional human user interface, e.g. a separate screen, and implementing its functionality in the human user interface module, enables for a compact set-up which is especially of advantage in crowded situations where a large number of further medical devices may be present.

According to an advantageous embodiment of the present invention, the oxygen storage and supply unit, especially the central control unit, is further adapted to provide the control signal to the valve control module on the basis of treatment data. These treatment data may include a target oxygen saturation in the blood of the patient, e.g. in form of a percent or an absolute value. Treatment data of this kind may be externally provided and may be transmitted to the oxygen storage and supply unit, e.g. wirelessly via a communications module.

For example to enable treatment data to be transmitted to the oxygen storage and supply unit, the oxygen storage and supply unit may be adapted to communicate with an external device like a stationary or handheld computer, a smartphone and/or a tablet PC. The oxygen storage and supply unit may also be adapted to be integrated into a communications network, especially into a cloud and/or a communications network defined by one or more network servers.

According to a preferred embodiment of the invention, the central control unit of the oxygen storage and supply unit or at least one sub-unit thereof may be programmed or programmable with a computer program comprising a treatment algorithm. On the basis of the treatment algorithm, the control unit may provide the control signal to the at least one outlet valve module. The treatment algorithm can be adapted to select different treatment regimens depending on patient specific data (e.g. disease, age, severity of disease, weight etc.) and/or further inputs like ambient temperature, altitude, atmospheric pressure, heart rate, breathing frequency, breathing volume etc. Using such additional inputs, oxygen delivery may further be tailored to the specific needs of the specific patient and/or the specific situation of the patient. A treatment regimen can include an amount of oxygen delivered to the patient.

According to a preferred embodiment of the present invention, the human user interface module of the oxygen storage and supply unit is adapted to process user input. For example, the human user interface module may include a capacitive and/or resistive touchscreen. The human user interface module may also include a screen and separate user input means, e.g. a keyboard or keypad. On the basis of the processed user input, the oxygen storage and supply unit, especially its central control module, may change operating parameters and/or parameters of the treatment algorithm and/or store corresponding data in a memory.

Advantageously, the human user interface module is adapted to process the treatment data mentioned above as user input. In this way, the oxygen storage and supply unit can be operated without having to communicate with other devices besides the oxygen saturation sensing unit. The communication abilities of the oxygen storage and supply unit can thus be limited to exactly this communication, significantly reducing the danger of network attacks by intruders. A direct user input of the treatment data at the human user interface module, and thus in some proximity of the patient, reduces possible errors caused by an abstraction from the real situation that may be introduced in a computer network for a user.

According to a particularly preferred embodiment of the present invention, however, the oxygen storage and supply unit, especially its central control unit, is further adapted to provide data to at least one external device. Such data may include the oxygen saturation value indicating an oxygen saturation of the blood of a patient corresponding processed data, e.g. a history of oxygen saturation values over a period of time and/or mean and/or average and/or extreme values of such oxygen saturation values. Also further data as mentioned above for the treatment algorithm, especially patient data, may be provided Additionally, the data relating to the oxygen storage and supply unit may be provided in this way.

The data relating to the oxygen storage and supply unit may, according to a particularly preferred embodiment of the invention, include a remaining oxygen supply time, an expiry date, a vessel type, a vessel location, an environmental temperature, an oxygen usage, a time since filling, a rate of oxygen usage, an oxygen pressure, an oxygen temperature, usage data, transportation data, and oxygen remaining in the vessel. Such data may be determined and/or provided by a pressure vessel monitoring module that may also be adapted to read information provided with the pressure vessel, e.g. as a barcode and/or a RFID tag. The remaining oxygen supply time, e.g., may be used to schedule attendance of staff to exchange or refill the oxygen storage and supply unit early enough, if necessary. The remaining oxygen supply time may be determined or estimated on the basis of a pressure of the oxygen remaining in the pressure vessel and on the basis of a recent or average oxygen consumption.

The oxygen storage and supply unit, especially its central control unit, may, according to a particularly preferred embodiment of the present invention, further be adapted to monitor patient data, the patient data including at least one of patient identity, patient type, blood pressure, heart rate, breathing frequency and/or intensity and oxygen usage by the patient. For monitoring such patient data, at least one patient data monitoring module may be provided. This at least one patient data monitoring module may be supplied with corresponding patient data e.g. via the communications module wirelessly receiving such data and/or via the human user interface module at which e.g. patient identity data like name, age or sex is entered by clinical staff.

The oxygen storage and supply unit preferentially further includes a memory module adapted to store any of the data mentioned above, either in unprocessed or processed form. To increase privacy, corresponding data, e.g. patient data, may also be stored in encrypted form. The memory module may also be adapted to store a history or protocol of any of the data mentioned above in a form that prevents manipulation, e.g. including hash tags and/or encryption. In this way, the data may be used to document proper patient treatment in case this should be contested.

According to a particularly preferred embodiment of the present invention, the oxygen storage and supply unit may also be provided with manual control means, especially a with a manually operable actuator, that is adapted to regulate the at least one outlet valve module or at least one valve thereof. This allows for a manual operation of the oxygen storage and supply unit in case the valve control module and/or the central control unit fails, e.g. due to power losses.

As mentioned, the invention also relates to a corresponding medical oxygen supply system. The medical oxygen supply system comprises an oxygen storage and supply unit and an oxygen delivery unit. The oxygen storage and supply unit comprises a pressure vessel adapted to store a volume of pressurized oxygen, at least one controllable outlet valve module adapted to adjust an oxygen flow from the pressure vessel, a valve control module adapted to regulate the at least one outlet valve module on the basis of a control signal, a human user interface module adapted to indicate data relating to the oxygen storage and supply unit, a central control unit, a communications module, and a housing module at least partially enclosing the at least one controllable outlet valve module, the valve control module, the central control unit, the communications module, and the human user interface module.

In the medical oxygen supply system according to the present invention, an oxygen saturation sensing unit is provided. The central control unit of the oxygen storage and supply unit is adapted to provide the control signal to the valve control module on the basis of an oxygen saturation value indicating an oxygen saturation of the blood of a patient which is transmitted to the oxygen storage and supply unit from the oxygen saturation sensing unit and which is received in the oxygen storage and supply unit via the communications module.

Such an oxygen supply system preferably comprises an oxygen storage and supply unit as explained in detail above and below. Regarding particular features and advantages of such an oxygen storage and supply unit, reference is made to these explanations. These also refer to the medical oxygen supply system.

The present invention also relates to a method of oxygen supply in which a corresponding oxygen storage and supply unit or an oxygen supply system is used. Also regarding particular features and advantages of such a method, reference is made to the explanations given above.

Further advantages of the present invention are explained with reference to the appended drawings which illustrate an embodiment of the present invention.

### Brief Description of the Drawings

Figure 1 schematically illustrates an oxygen storage and supply unit according to a preferred embodiment of the present invention.
Figure 2 schematically illustrates an oxygen supply system according to a preferred embodiment of the present invention.

In the figures, elements with comparable function are indicated with identical reference numerals. Repeated explanations will be omitted for conciseness.

### Embodiments of the Invention

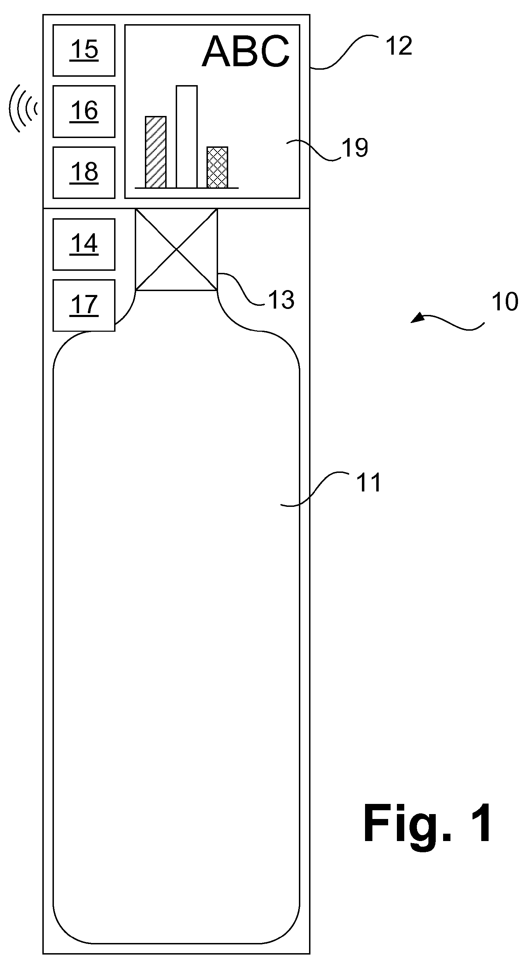

In figure 1, an oxygen storage and supply unit according to a preferred embodiment of the present invention is shown and designated 10.

The oxygen storage and supply unit 10 as shown in Figure 1 comprises a pressure vessel 11 adapted to store a volume of pressurized oxygen. The pressure vessel 11 is fluidly connected with a valve module 13, the valve module 13 being adapted to regulate a flow of oxygen from the pressure vessel 11 to an oxygen outlet. A valve control module 14 is provided, the valve control module 14 being adapted to control the valve 13 on the basis of a control signal from e.g. a central control unit 15. A housing 12 is provided, the housing 12 at least partially enclosing the shown elements.

The central control unit 15 may receive input signals via a communications module 16, the communications module 16 being adapted for wired and/or wireless communication with other communications modules (not shown in figure 1). The central controller 15 may, via the communications module 16 or directly, e.g. via a wired connection (not shown), receive diagnostic signals from a pressure vessel monitoring module 17.

The pressure vessel monitoring module 17 may monitor data relating to the pressure vessel 11 and provide such data to the central control unit 15. These data may include e.g. vessel identification data, a remaining oxygen supply time, an expiry date, a vessel type, a vessel location, an environmental temperature, an oxygen usage, a time since filling, a rate of oxygen usage, an oxygen pressure, an oxygen temperature, usage data, transportation data, and oxygen remaining in the vessel, as mentioned above. Some or all of these data may also be obtained in the central control unit 15 on the basis of raw data, e.g. sensor data, provided by the pressure vessel monitoring module 17, or without support from the pressure vessel monitoring module 17. The pressure vessel monitoring module 17 may also be included in the central control unit 15.

Besides the pressure vessel monitoring module 17, one or more additional monitoring modules may be provided (not shown), e.g. adapted to monitor further data and provide such data to the central control unit 15. Optionally, the one or more further monitoring modules may be included in the central control unit 15. For example, further data may include data relating to the operating environment of the oxygen storage and supply unit 10, like location, smoke, temperature, movement or vibration.

The oxygen storage and supply unit 10 further includes a patient data monitoring module 18, the patient data monitoring module 18 being adapted to monitor, e.g. wirelessly via the communications module 16 or alternatively via a wired connection, patient data and report such data to the central controller 15. The patient data monitoring module 18 may also at least in part be included in the central controller 15. Data that may be monitored by the patient data monitoring module 18 include, according to the embodiment of the invention as shown in figure 1, oxygen saturation, and may also include patient identity, patient type, blood pressure, heart rate, breathing and oxygen usage by the patient.

Each of the modules 14, 16, 17 and 18 of the oxygen storage and supply unit 10 as shown in figure 1 may be operably interconnected and/or operably connected to the central controller 15 via wired connections (not shown) or wirelessly via the communications module 16. As mentioned, functions of the modules 14, 16, 17 and 18 may also at least partially integrated in the central controller 15 and/or in other modules. The oxygen storage and supply unit 10 as shown in figure 1 may also include a global positioning system (GPS) module (not shown) and optionally a transmitter (not shown), the latter being adapted to broadcast the location of the oxygen storage and supply unit 10, e.g. via the communications module 16.

A human interface module 19, e.g. a display or a touchscreen, may be provided. Alternatively, separate human interface modules, e.g. for separate display and user input, may be provided. The human interface module 19 may be supplied by the central controller 15 by corresponding output data for display. The human interface module 19 may also be adapted to provide user input to the central controller 15

The central controller 15 and/or the valve control unit 14 may also be programmed to respond to one or more automatic valve closure commands transmitted thereto upon receipt of an adverse situation signal generated by detection of any one or more of smoke, adverse vibration, adverse movement, adverse temperature, excessive or non-programmed oxygen usage, excessive or unexpected oxygen pressure, oxygen dispensing in the absence of patient data from the monitoring module 18, passage of an expiry date, unexpected or non-programmed location of the oxygen storage and supply unit 14, absence of one or more signals from the central controller 15, from one ore more of the monitoring modules 17, 18, from the communications module 16 or from the human interface module 19.

The oxygen storage and supply unit 10 may also be adapted, especially via the central controller 15, to perform an automatic check and restart function for periodically checking received data after closure of the valve 13 due to detection of the adverse situation signal as mentioned, and for re-starting gas flow in the absence of receipt of a further adverse situation signal. The automatic check and restart function may include an intelligent restart function for allowing oxygen to be delivered in accordance with a pre-determined control strategy. Also an external server or computer (not shown in figure 1) may be used to implement these functions.

The oxygen storage and supply unit 10 may also be adapted, especially via the central controller 15, to perform an operable mode in which it is fully operational and a sleep mode in which it is not fully operable but is able to be placed back into the operable mode (resumed) upon receipt of a signal, e.g. a signal from one ore more of the monitoring modules 17, 18 and/or from the communications module 16. Especially in absence of such a signal for more than a predetermined period of time, the central controller 15 may enter the sleep mode, especially to save energy provided by an energy module, e.g. a battery (not shown). However, it will be appreciated that a signal for entering or resuming from sleep mode may also be sent from any other suitably functional device. Such suitable other devices include the human interface module 19.

The human interface module 19 may be adapted to provide information or warnings to a user or accepting command inputs from a user. Preferably, said human interface module 19 has multiple modes of operation and the central controller 15 may be operable to alter the mode of operation of the interface module 19, especially dependent upon a signal from one ore more of the monitoring modules 17, 18 and/or from the communications module 16. Such a signal may comprise a location signal which, upon receipt by the central controller 15 causes the triggering of a change of state of one or other or both of the valve position of the valve 12 and/or the status of the human interface module 19. The valve position of the valve 12 may be altered such as to prevent or allow or restrict or control oxygen delivery when said oxygen storage and supply unit 10 is in a particular location or absent from a location. The human interface module 19 may be altered such as to enable or disable audio alarms, mute such alarms, enable or disable visual alarms and/or reduce light output from such alarms. Any audio or visual alarms or displays may be provided separately to other portions of the human interface module 19 and may comprise discrete alarms or displays positioned at convenient locations on the oxygen storage and supply unit 10 or in close proximity thereto. The human interface module 19 may be operable to change or cancel audio output, visual output, operational command output, user access capability, user interface capability and/or operational capability. The human interface module 19 may also be operable to receive manually input patient data or treatment data and for displaying such data in an appropriate form to a member of hospital staff, the patient or another person.

The oxygen storage and supply unit 10 or its central controller 15 may also include a memory (not shown) for retrievably storing any one or more of the data as mentioned above, especially a history or protocol of such data. This memory may be accessible by the human interface 19 and/or the communications module 16, such as to allow hospital staff to e.g. view patient data such as to make treatment decisions based thereon. Such an arrangement is of particular use when the memory is incorporated in association with the oxygen storage and supply unit 10 such that it moves together with the oxygen storage and supply unit 10. This will allow hospital staff to quickly and easily read the patient data of an incoming or new patient and integrate that data into any patient management or any patient treatment programme. The oxygen storage and supply unit 10 or its central controller 15 may also be operable to transmit any one or more of the data as indicated above to a further device (not shown), the human interface 19 or a computer system (not shown) for subsequent analysis thereon by any one of a number of members of staff. Transmission may preferably performed in wireless form via the communications module 16.

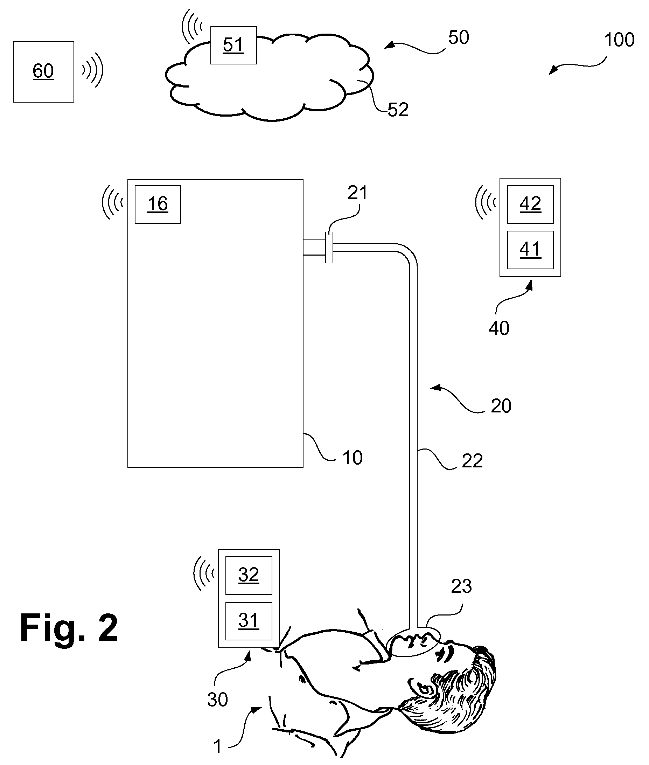

In figure 2, a medical oxygen supply system according to a preferred embodiment of the present invention is shown and designated 100. The medical oxygen supply system 100 may include an oxygen storage and supply unit 10 as shown in Figure 1.

An oxygen outlet of the oxygen storage and supply unit 10, which is not shown in detail in figure 2, is coupled to an oxygen supply unit 20 via a coupling module 21, the coupling module e.g. comprising coupling means adapted to provide a screwed or bayonet coupling to the oxygen supply unit 20. The oxygen supply unit 20 further comprises an oxygen supply line 22, the oxygen supply line 22 extending between the coupling module 21 and a patient module 23. In the example shown, the patient module 23 comprises a facial mask, but other suitable means of oxygen supply to a patient 1 may be provided, e.g. a nasal cannula.

The patient 1 is further equipped with an oxygen saturation sensing unit 30 which may operate according to the principles as explained above for an oximeter. The oxygen saturation sensing unit 30 may comprise an oxygen saturation sensing module 31 adapted to provide an oxygen saturation value of a blood of a patient 1 when the oxygen saturation sensing unit 30 is connected to the patient 1.

In the example shown in figure 2, the oxygen saturation sensing unit 30 further comprises a communications module 32 which is adapted to wirelessly communicate the oxygen saturation value, or a signal based thereon, at least to the communications module 16 of the oxygen storage and supply unit 10. However, also a wired connection is possible, such a wired connection also allowing an oxygen saturation sensing unit 30 to be supplied with electric energy.

In operation, the oxygen saturation sensing unit 30, via its communications module 32, may communicate the oxygen saturation value, or the signal based thereon, to the communications module 16 of the oxygen storage and supply unit 10. On the basis of the oxygen saturation value, or the signal based thereon, the oxygen storage and supply unit 10 or its central control unit 15 may, e.g. based on treatment data stored in the central control unit 15, an user input via the human interface module 19, or on the basis of a signal from a further device, provide a signal to the control module 14 of the valve 13, in order to provide a predetermined oxygen flow to the patient 1.

The further device mentioned in connection with figure 1 is indicated 40 in figure 2. It may be, as mentioned, mounted on the cylinder itself or may be a freestanding computer having a display, a hand-held computer, a mobile telephone or any such similar device. In the embodiment shown in figure 2, the further device 40 may include an user interface module 41 and a communications module 42, as commonly the case for corresponding devices. Also the communication between the further device 40, or its communications module 42, and the oxygen storage and supply unit 10, or its communications module 16, may be by means of wired or wireless connections. The further device 40 and/or the human interface system 19 may be provided with a software application ("app") to facilitate human interaction including display of information and modification or control through human input. The further device 40 and the oxygen saturation sensing unit 30, via their communication modules 32 and 42, may also communicate with each other, either directly or via the oxygen storage and supply unit 10 or its communications module 16 or via a central server unit 50.

The central server unit 50, preferably including a server 51 and/or a cloud 52, may be adapted to communicate with one or more of the units 10 or 30 or the further device 40 or their communications units 16, 32 or 42 in wired or wireless form and/or to store or provide data, treatment protocols and user inputs as mentioned above. A server 51 may be a mainframe computer in a hospital to a cloud based server. Either arrangement of the server unit 50 may be operably connected to a management unit 60 for allowing management of the oxygen storage and supply unit 10 or the medical oxygen supply system 100 as and when desired.

Such a management unit 60 may further include input or output devices such as a keyboard or a screen and may be linked to any of the units shown, e.g. by wireless links. Typical management activities performed by the management unit 60 may include controlling certain types of oxygen storage and supply units 10 such that they are not able to dispense gas in certain locations or when outside of certain locations, restricting or stopping the supply of gas from cylinders during certain times, emergency shut-down of cylinders in the event of fire or other dangerous situations, initiating audio or visual alarms on cylinders, communicating with hospital staff or patients through the human interface 19 or in other ways or via alarms, providing location information relating to one or more oxygen storage and supply units 10 to staff, ordering replacement oxygen storage and supply units 10 or their pressure vessels 11 in advance of gas expiry, disablement of oxygen storage and supply units 10 if their pressure vessels 11 are past their expiry date, etc.

It will be appreciated that the communications module 16 is crucial to many aspects of the present invention as it acts as the communicator between units 10, 30, and preferable units 40, 50 and 60.

## Claims

**1.** An oxygen storage and supply unit (10) comprising a pressure vessel (11) adapted to store a volume of pressurized oxygen, at least one controllable outlet valve module (13) adapted to adjust an oxygen flow from the pressure vessel (11), a valve control module (14) adapted to regulate the at least one outlet valve module (13) on the basis of a control signal, a human user interface module (19) adapted to indicate data relating to the oxygen storage and supply unit (10), a central control unit (15), a communications module (16), and a housing module (12) at least partially enclosing the at least one controllable outlet valve module (13), the valve control module (14), the central control unit (15), the communications module (16), and the human user interface module (19), **characterized in that** the oxygen storage and supply unit (10) is adapted to provide the control signal to the valve control module (14) on the basis of an oxygen saturation value indicating an oxygen saturation of the blood of a patient (1) which is transmitted to the oxygen storage and supply unit (10) and which is received in the oxygen storage and supply unit (10) via the communications module (16).

**2.** The oxygen storage and supply unit (10) according to claim 1, wherein the communications module (16) is adapted for wireless communication according to at least one wireless communication protocol.

**3.** The oxygen storage and supply unit (10) according to any one of the preceding claims, further adapted to provide user information via the human user interface module (19) on the basis of the oxygen saturation value transmitted to the oxygen storage and supply unit (10).

**4.** The oxygen storage and supply unit (10) according to any one of the preceding claims, further adapted to provide the control signal to the valve control module (14) on the basis of treatment data.

**5.** The oxygen storage and supply unit (10) according to claim 4, wherein the human user interface module (19) is adapted to process user input.

**6.** The oxygen storage and supply unit (10) according to claim 5, wherein the human user interface module (19) is adapted to process the treatment data as user input.

**7.** The oxygen storage and supply unit (10) according to any one of the preceding claims, wherein the central control unit (15) is programmed or programmable with a treatment algorithm adapted to select a treatment regimen depending on patient specific data including at least one of disease, age, severity of disease, weight, heart rate, breathing frequency and breathing volume and/or at least one further input selected from ambient temperature, altitude and atmospheric pressure.

**8.** The oxygen storage and supply unit (10) according to any one of the preceding claims, further adapted to provide data to at least one external device (40).

**9.** The oxygen storage and supply unit (10) according to any one of the preceding claims, the data relating to the oxygen storage and supply unit including at least one of a remaining oxygen supply time, an expiry date, a vessel type, a vessel location, an environmental temperature, an oxygen usage, a time since filling, a rate of oxygen usage, an oxygen pressure, an oxygen temperature, usage data, transportation data, and oxygen remaining in the vessel.

**10.** The oxygen storage and supply unit (10) according to any one of the preceding claims, further being adapted to monitor patient data, the patient data including at least one of patient identity, patient type, blood pressure, heart rate, breathing frequency and/or intensity and oxygen usage by the patient.

**12.** The oxygen storage and supply unit (10) according to any one of the preceding claims, further including a memory module.

**13.** An oxygen supply system (100) comprising an oxygen storage and supply unit (10) and an oxygen delivery unit (20), the oxygen storage and supply unit (10) comprising a pressure vessel (11) adapted to store a volume of pressurized oxygen, at least one controllable outlet valve module (13) adapted to adjust an oxygen flow from the pressure vessel (11), a valve control module (14) adapted to regulate the at least one outlet valve module (13) on the basis of a control signal, a human user interface module (19) adapted to indicate data relating to the oxygen storage and supply unit (10), a central control unit (15), a communications module (16), and a housing module (12) at least partially enclosing the at least one controllable outlet valve module (13), the valve control module (14), the central control unit (15), the communications module (16), and the human user interface module (19), **characterized in that** an oxygen saturation sensing unit (30) is provided and **in that** the oxygen storage and supply unit (10) is adapted to provide the control signal to the valve control module (14) on the basis of an oxygen saturation value indicating an oxygen saturation of the blood of a patient (1) which is transmitted to the oxygen storage and supply unit (10) from the oxygen saturation sensing unit (30) and which is received in the oxygen storage and supply unit via the communications module.

**14.** An oxygen supply system (100) according to claim 13, **characterized by** an oxygen storage and supply unit (10) according to any one of claims 1 to 12.

**15.** A method of medical oxygen supply, **characterized in that** an oxygen supply system (100) according to claim 13 or 14 or an oxygen storage and supply (10) according to any one of claims 1 to 12 is used in the method.
